# EUROPEAN PATENT APPLICATION

(11) **EP 4 115 899 A1**
(43) Date of publication of application: **11.01.2023**
(21) Application number: 21761756.2
(22) Date of filing: 19.02.2021
(51) Int. Cl.: A61K 38/18, G01N 33/53, A61P 9/04

(54) **METHOD FOR PREVENTING, TREATING OR DELAYING HEART FAILURE BY USING NEUREGULIN, AND COMPOSITION**

(30) Priority: 24.02.2020 CN 202010113391; 13.03.2020 CN 202010174086
(71) Applicant: Zensun (Shanghai) Science & Technology, Co., Ltd., Shanghai 201203 (CN)
(72) Inventor: ZHOU, Mingdong, Shanghai 201203 (CN)
(74) Representative: Jones Day
(86) International application number: PCT/CN2021/076772
(87) International publication number: WO 2021/169845

(57) **Abstract**

The present invention provides an application of neuregulin protein in the preparation of drugs for preventing, treating or delaying human heart failure, and neuregulin protein for use in a method for preventing, treating or delaying human heart failure. The method comprises: testing a patient first before treatment, the testing comprises detecting the plasma level of NT-proBNP or BNP; and then providing appropriate treatment on the basis of the test result. When the test result is within an optimal treatment range, the patient is suitable for the treatment of heart failure by administering an effective dose of neuregulin.

## Description

### FIELD OF THE INVENTION

The present invention relates to an application of Neuregulin in the manufacture of medications for preventing, treating or delaying human heart failure and the method for the administration of the said medications therefor. In particular, the present invention provides a method for preventing, treating or delaying human heart failure by administering medications comprising neuregulin to a specific population of patients with heart failure. In particular, the present invention provides a method for treating heart failure using medications comprising neuregulin. The said method involves pre-treatment testing and determination of appropriate treatment for an patient based on the test results. It is appropriate to administer an effective dose of Neuregulin to an patient with heart failure when pre-treatment test results are in an optimal therapeutic range.

### BACKGROUND OF THE INVENTION

Heart failure (HF) is a syndrome associated with cardiac insufficiency caused by a variety of heart diseases. As predominant treatments for patients with HF, angiotensin-converting enzyme (ACE) inhibitors are effective in dilating blood vessels, lowering blood pressure and reducing cardiac load. Despite the statistically significant reduction in mortality (%) in patients treated with ACE inhibitors, the actual reduction in mortality by only an average of 3%-4%, with some potential side effects. Other prophylactic or therapeutic options for HF also have limitations. For instance, heart transplantation is inevitably more costly and more invasive than medications and also restricted by the availability of heart donors. Treatment options with mechanical devices such as biventricular pacemaker are also invasive and costly. Due to the shortcomings of existing therapies, new treatments are urgently needed.

A promising new treatment involves the administration of neuregulin (NRG, Neuregulin, heregulin (HRG)), also known as glial growth factor (GGF), or new differentiation factor (NDF), a glucoprotein with a molecular weight of 440KD, to patients with HF or at risk of HF; NRGs are proteins involved in intercellular signaling, which act as ligands bound to the ErbB family of receptor tyrosine kinases. The family of NRGs consists of 4 members: NRG1, NRG2, NRG3, and NRG4 (Falls et al., Exp Cell Res.284:14-30,2003). NRGs are involved in a range of biological responses: stimulating the differentiation of breast cancer cells and the production of milk proteins, inducing the differentiation of neural crest cells into Schwann cells, stimulating the synthesis of acetylcholine receptors in skeletal muscle cells, and promoting the survival of myocardial cells and the synthesis of DNA. Results from in vivo studies in embryos of homozygous mice with severe Neuregulin deficiency showed Neuregulin was essential for cardiac and neural development. NRG1 plays an important role in the development of nervous system, heart and mammary gland; some evidences suggest that NRG1 signaling plays a role in the development and functioning of some other organs/system and in the pathogenesis of human diseases (such as schizophrenia and breast cancer). NRG1 exists in multiple isoforms. Results from studies in gene mutant mice (geneknockout mice) suggested that the in vivo functions of isoforms vary with their N-terminus regions or epidermal growth factor (EGF) similar regions. The present invention is based on neuregulin 1β (NRG-1β).

Neuregulin 1β is a transmembrane protein (Holmes et al.,Science 256,1205-1210,1992). The extramembrane portion is N terminus, including Ig-like domain and EGF-like domain, intramembrane portion is C terminus. Under the action of the metalloprotease of extracellular matrix, the extramembrane portion of neuregulin can be cut off by enzymes and get into a free state, thereby facilitating neuregulin's binding to ErbB receptors on the surface of surrounding cells and activating the corresponding cell signaling.

The ErbB family also has four members, i.e., ErbB1, ErbB2, ErbB3 and ErbB4, all of which are transmembrane proteins with a molecular weight around 180-185 KD. With the exception of ErbB2, they all comprise ligand-binding domain at their extramembrane N terminus; with the exception of ErbB3, their intramembrane C terminus all has the activity of protein tyrosine kinase. ErbB1 is a epidermal growth factor (EGFR) receptor, while ErbB3 and ErbB4 are neuregulin receptors. Of these neuregulin receptors, only ErbB2 and ErbB4 are highly expressed in the heart (Yarden et al.,Nat Rev Mol Cell Bio1,2:127-137,2001).

Binding of neuregulin to the extramembrane portion of ErbB3/ErbB4 may lead to heterodimerization of with ErbB3/ErbB4 with other ErbB family members (often including ErbB2), or homodimerization of ErbB4 itself, which is responsible for the phosphorylation of the intramembrane portion (Yarden et al.,Nat Rev Mol Cell Biol,2:127-137,2001). The phosphorylated intramembrane portioni can further bind to multiple intracellular signaling proteins, thereby activating downstream ERK or AKT signaling pathways, giving rise to a series of cellular responses: including stimulating or inhibiting the proliferation, apoptosis, migration, differentiation or cytoadherence of cells.

Neuregulin is especially essential for heart development (WO0037095, CN1276381, WO03099300, WO9426298, US6444642, WO9918976, WO0064400, Zhao et al.,J.Biol.Chem.273,10261-10269,1998). In early embryonic development, the expression of neuregulin is mainly limited to endocardium; it is then released into surrounding myocardial cells via paracrine pathway and bound to the extramembrane portion of protein tyrosine kinase receptor ErbB4 on the cell membrane, and ErbB4 then forms heterodimer with ErbB2. The formation and activation of ErbB4/ErbB2 complex are indispensable for the forming of cardiac trabeculae in early spongiform heart. The missing of any gene of neuregulin, ErbB4 and ErbB2 will lead to the absence of trabeculae in and the death of fetus in uterus during early development. According to WO0037095, neuregulin at certain concentrations can continuously activate the ERK signaling pathway, promote the growth and differentiation of myocardial cells, guide the reconstruction of myocomma and cell framework in myocardial cells and at cytoadherence, improve the structure of myocardial cells, strengthen the contraction of myocardial cells. Furthermore, it was stated in WO0037095 and WO003099300 that neuregulin can be used for the detection, diagnosis and treatment of numerous cardiovascular diseases.

Listed below are some prior art technical literature related to the present invention: 1.Cardiac muscle function and manipulation:WO0037095, 2. New applications of growth factor neuregulin and its analogs : CN1276381 ; 3.Neuregulin based methods and composition for treating cardiovascular diseases:WO03099300 ; 4.Zhao YY,Sawyer DR,Baliga RR,Opel DJ,Han X,Marchionni MA and Kelly RA.Neuregulins Promote Survival and Growth of Cardiac Myocytes.J.Biol.Chem.273,10261-10269(1998) ; 5.Methods for treating muscle diseases and disorder:WO9426298; 6.Methods of increasing myotube formation or survival or muscle cell mitogenesis,differentiation or survival using a neuregulin:US6444642.7.Therapeutic methods comprising use of a neuregulin:WO9918976 ; 8.Methods for treating congestive heart failure:WO0064400; 9.Holmes WE,Sliwkowski MX,Akita RW,Henzel WJ,Lee J,Park JW,Yansura D,Abadi N,Raab H,Lewis GD,et al.Identification of heregulin,a specific activator p185erbB2.Science 256,1205-1210(1992) ; 10.Falls DL.Neuregulins:functions,forms and signalingstrategies.Experimental Cell Research,284,14-30(2003).11.Yarden Y,Sliwkowski X.Untangling the ErbB signaling Network.Nature Reviews:Molecular Cell Biology,2127-137(2001).

Studies have shown that the EGF-like domain of NRG1 has approximately 50 to 64 amino acids, adequate to bind to and activate these receptors. Previous studies showed Neuregulin-1β (NRG-1β) can directly bind to ErbB3 and ErbB4 with high affinity. Orphan receptor ErbB2 can form heterodimers with ErbB3 or ErbB4 with affinity higher than the affinity of ErbB3 or ErbB4 for forming homodimer. Results from neurodevelopment studies suggested that the formation of sympathetic nervous system requires complete NRG-1β, ErbB2 and ErbB3 signaling system. Targeted destruction of NRG-1β, or ErbB2 or ErbB4 could be lethal to fetus due to the resulting developmental defect of the heart. A recent study also highlighted the important role of NRG-1β, ErbB2 and ErbB4 in the development of the cardiovascular system and the maintenance of the normal cardiac function of adults. It was showed that NRG-1β could strengthen the tissue structure of sarcomere of adult myocardial cells. The administration of a recombinant NRG-1β EGF-like domain could significantly improve or prevent the degeneration of myocardial function in a variety of HF animal models and similar results were observed in clinical studies. However, more studies are needed to make clear how to use this product or composition and how to achieve better therapeutic effect in some subpopulations. In the present invention, a method for the treatment of heart failure with Neuregulin-containing medications is provided. The said method involves pretreatment testing and determination of appropriate treatment for an patient based on the test results. It is appropriate to administer an effective dose of Neuregulin to an patient with heart failure when pre-treatment test results are in an optimal therapeutic range.

### SUMMARY OF THE INVENTION

### A. Brief Summary

During the clinical study on the treatment of HF with Neuregulin, the applicant discovered that Neuregulin therapy achieved significant therapeutic effect in HF patients who were screened by grading according to New York Heart Association (NYHA) Functional Classification or by measuring the NT-proBNP or BNP levels in patient plasma. These therapeutic results included significantly decreased mortality. These therapeutic results included significantly decreased readmissions for treatment. Moreover, studies showed that NRG significantly improved or prevented the deterioration of myocardial function in various animal models of HF and patients with HF. Neuregulin, Neuregulin polypeptides, Neuregulin variants, Neuregulin derivatives, compositions with NRG-like functional domain(s) or compounds with Neuregulin similar activity are all in the scope of this invention.

In the first aspect of the present invention, a drug composition that comprises neuregulin at effective dose for the treatment of heart failure is provided. Patients who received treatment with that drug composition had achieved significant therapeutic results. In some embodiments, the therapeutic effect manifested as significantly decreased mortality. In some embodiments, the therapeutic effect manifested as significantly decreased readmissions for treatment. In some embodiments, the therapeutic effect manifested as decreased levels of biomarkers, indicating improvement of chronic heart failure. In some embodiments, the drug composition was administered to patients for an introduction regimen. In some embodiments, the introduction regimen included repeated administration of the drug composition for at least 3, 5, 7 or 10 consecutive days. In some preferred embodiments, the administration of the drug composition was maintained for at least 3, 6 or 12 months after the introduction regimen.In some preferred embodiments, the maintenance regimen included the administration of the drug composition every 3, 5, 7 or 10 days. In a preferred embodiment, the treated HF patients were females. In a preferred embodiment, the treated HF patients were female HF patients whose NT-proBNP level in plasma was not more than 3000fmol/ml and male HF patients whose NT-proBNP level in plasma was not more than 1600fmol/ml. In a preferred embodiment, the treated HF patients were female HF patients whose NT-proBNP level in plasma was not more than 3000fmol/ml.

In another aspect of the present invention, a method is provided for improving the survival rate or lowering the mortality rate of chronic HF patients, and the method includes the administration of a drug composition comprising neuregulin at effective dose to chronic HF patients. In some embodiments, the drug composition was administered to patients for an introduction regimen. In some preferred embodiments, the introduction regimen included repeated administrations of the drug composition for at least 3, 5, 7 or 10 consecutive days. In some preferred embodiments, the administration of the drug composition was maintained for at least 3, 6 or 12 months after the introduction regimen. In some preferred embodiments, the maintenance regimen included the administration of the drug composition every 3, 5, 7 or 10 days. In a preferred embodiment, the treated HF patients were females. In a preferred embodiment, the treated HF patients were female HF patients whose NT-proBNP level in plasma was not more than 3000fmol/ml and male HF patients whose NT-proBNP level in plasma was not more than 1600fmol/ml. In a preferred embodiment, the treated HF patients were female HF patients whose NT-proBNP level in plasma was not more than 3000fmol/ml.

In another aspect of the present invention, information on the application of Neuregulin in drug preparation is provided. The drug can provide long-term benefit to chronic heart failure patients. In an embodiment, long-term benefit means improved survival. In an embodiment, long-term benefit means lowered re-admission rate. In another embodiment, long-term benefit denotes improved (lowered) biomarkers that can predict the long-term prognosis of HF. In some embodiments, the drug was administered to patients for an introduction regimen. In some preferred embodiments, the introduction regimen included repeated administrations of the drug for at least 3, 5, 7 or 10 consecutive days. In some preferred embodiments, patients received maintenance administration for at least 3, 6 or 12 months after the introduction regimen. In some preferred embodiments, the maintenance regimen included the administration of the drug every 3, 5, 7 or 10 days. In a preferred embodiment, the treated HF patients were females. In a preferred embodiment, the treated HF patients were female HF patients whose NT-proBNP level in plasma was not more than 3000fmol/ml and male HF patients whose NT-proBNP level in plasma was not more than 1600fmol/ml. In a preferred embodiment, the treated HF patients were female HF patients whose NT-proBNP level in plasma was not more than 3000fmol/ml.

In another aspect of the present invention, a method for screening HF patients suitable for treatment with Neuregulin is provided. The method includes measuring the patient's NT-proBNP plasma level. In an embodiment, it was demonstrated that a NT-proBNP plasma level not more than 4000fmol/ml indicated the HF patient was suitable for treatment with Neuregulin. In another embodiment, an NT-proBNP plasma level in the range of 1600fmol/ml - 4000fmol/ml indicated that the patient's HF was suitable for treatment with Neuregulin. In another embodiment, an NT-proBNP plasma level not more than 1600fmol/ml indicated that the patient's HF was suitable for treatment with Neuregulin. In another preferred embodiment, it was shown that the HF in female HF patients who had a NT-proBNP plasma level not more than 3000fmol/ml were suitable for treatment with Neuregulin. In another preferred embodiment, it was shown that the female HF patients who had an NT-proBNP plasma level not more than 3000fmol/ml and male HF patients who had an NT-proBNP plasma level not more than 1600fmol/ml were suitable for treatment with Neuregulin. In another preferred embodiment, it was shown that female HF patients were suitable treatment with Neuregulin regardless of their NT-proBNP plasma level.

In another aspect of the present invention, a method for screening HF patients suitable for treatment with Neuregulin is provided. The method includes the evaluation of patient's cardiac function in accordance with New York Heart Association (NYHA) Functional Classification. In an embodiment, NYHA Class II indicated that the patient's HF was suitable for treatment with Neuregulin. In another embodiment, NYHA Class III indicated that the patient's HF was suitable for treatment with Neuregulin.

In another aspect of the present invention, a method was provided for the treatment of chronic heart failure with Neuregulin. The method includes pretreatment evaluation procedures and determination of patient's eligibility for receiving treatment with Neuregulin according to the evaluation results. In some embodiments, the evaluation procedures included the grading of chronic HF patient's NYHA function class. In another embodiment, the evaluation procedures included the determination of every chronic HF patient's plasma NT-proBNP or BNP level.

In another aspect of the present invention, a diagnostic reagent kit is provided for the screening of HF patients who are suitable for treatment with Neuregulin. In an embodiment, the reagent kit comprises immunoassay reagent(s) for measuring HF patient's NT-proBNP plasma level. A level not more than 4000fmol/ml indicates that the patient is suitable for treatment of HF with Neuregulin. In another embodiment, an NT-proBNP plasma level in the range of 1600fmol/ml - 4000fmol/ml indicated that the patient's HF was suitable for treatment with Neuregulin. In another embodiment, an NT-proBNP plasma level not more than 1600fmol/ml indicated that the patient's HF was suitable for treatment with Neuregulin. In another preferred embodiment, it was shown that the HF in female HF patients who had a NT-proBNP plasma level not more than 3000fmol/ml were suitable for treatment with Neuregulin.

In another aspect of the present invention, a concomitant diagnostic test is provided for the treatment of chronic HF with Neuregulin. N-terminal brain natriuretic peptide (NT-proBNP) is used as a biomarker for the diagnostic test. In some embodiments, a level not more than 4000fmol/ml indicated that the patient's HF was suitable for treatment with Neuregulin. In another embodiment, a level of 1600fmol/ml to 4000fmol/ml indicated that the patient's HF was suitable for treatment with Neuregulin. In another embodiment, an NT-proBNP plasma level not more than 1600fmol/ml indicated that the patient's HF was suitable for treatment with Neuregulin. In another preferred embodiment, it was shown that the HF in female HF patients who had a NT-proBNP plasma level not more than 3000fmol/ml were suitable for treatment with Neuregulin.

In another aspect of the present invention, a concomitant diagnostic kit is provided to determine an HF patient's eligibility to receive treatment with Neuregulin. The concomitant diagnostic kit comprises a reagent kit for determining plasma NT-proBNP or BNP level and Instructions for Use to determine patient's eligibility to receive Neuregulin therapy according to the test results.

In another aspect of the present invention, a kit is provided. The kit includes one or more containers of Neuregulin at effective dose. The protein can be provided alone or combined with other compatible drugs/substances to form a composition. The preferred drug dosage form can be used in combination with sterile physiological saline, glucose solution, buffer solution or other compatible sterile solutions. Optionally, the composition can be lyophilized or dried. In an embodiment, the kit in this invention further includes a needle or syringe, preferably in a sterile package, for injection, and/or a packaged alcohol cotton ball. In another embodiment, the kit in this invention further includes Instructions for Use which, depending on the circumstances, may have instructions to doctors or patients on the use method of the drug composition. In another preferred embodiment, the kit in this invention comprises NRG at effective dose and reagent kit, the diagnostic kit comprises immunoassay reagent(s) for measuring HF patient's NT-proBNP plasma level.

In another aspect of the present invention, neuregulin is administered at effective dose to chronic HF patient who has a NT-proBNP plasma level in appropriate range for the therapy before treatment. In an embodiment, the appropriate range for therapy was not more than 4000fmol/ml. In another embodiment, the appropriate range for therapy was between 1600fmol/ml and 4000fmol/ml. In another embodiment, the appropriate range for therapy was not more than 1600fmol/ml. In another preferred embodiment, the appropriate range for female HF patients to receive therapy was not more than 3000fmol/ml. In another preferred embodiment, the plasma level was determined by immunoassay.

In another aspect of the present invention, neuregulin is administered at effective dose to chronic HF patients, whose cardiac function is graded according to New York Heart Association (NYHA) Functional Classification. In some embodiments, the specific functional class was NYHA Class II. In some embodiments, the specific functional class was NYHA Class III.

### B. Definitions

Unless otherwise defined, all technology terms used herein have the same meaning as is understood by general technicians in the art of the invention. All patent documents, patent application documents, published patent documents and other publications are incorporated herein by reference in their entirety. Where a definition described herein is not in agreement with or on the contrary to the corresponding definition in any of the above-mentioned references, the definition herein shall prevail.

Unless clearly indicated in the context, all single and plural determiners/nouns (e.g., a, an, the, these, etc.) are interchangeable, 'at least one' and 'one or more" are interchangeable.

The "neuregulin" or "NRG" used herein refers to proteins or polypeptides that can bind to and activate ErbB2, ErbB3, ErbB4 or their heterodimers or homodimers, including neuregulin isoforms, EGF-like domains of neuregulin, polypeptides comprising neuregulin EGF-like domain(s), neuregulin variants or derivatives and other neuregulin-like gene products capable of activating the above-mentioned receptors. Neuregulins also include NRG1, NRG2, NRG3 and NRG4 proteins, polypeptides, fragments and complexes with NRG-like functions. Preferably, neuregulins are proteins or polypeptides capable of binding to and activating ErbB2/ErbB4 or ErbB2/ErbB3 heterodimers. To cite an illustrative rather than restrictive example, the neuregulin of the present invention is a fragment of NRG-1β2 isoform, i.e., the fragment of amino acids at Position 177-Position 237 which comprises an EGF-like domain. The fragment has the following amino acid sequence: SHLVKCAEKEKTFCVNGGECFMVKDLSNPSRYLCKCPNEFTGDRCQNYVMASFYKAEEL YQ (SEQ ID NO:1). The neuregulin used in this invention can activate the above-mentioned receptors and regulate their biological functions; for example, it can stimulate the synthesis of acetylcholine receptors in skeletal muscle cells, promote the differentiation, survival and DNA synthesis of myocardial cells. Neuregulins also include those neuregulin variants that have conservative mutations that do not substantially affect the protein's biological functions. As wellappreciated by general technicians in the art, a single amino acid mutation in a non-critical region generally does not change the biological functions of the protein or polypeptide (see Watson et. al."Molecular Biology of the Gene,4th Edition,1987,The Bejacmin/Cummings Pub.co.,p.224). The neuregulin used in the present invention can be isolated from natural sources, or obtained through genetic recombination, artificial synthesis or other approaches.

The "epidermal growth factor-like domain" or "EGF-like domain" used herein refer to polypeptide fragments which are encoded by neuregulin gene and capable of binding to and activating ErbB2, ErbB3, ErbB4 or their heterodimers or homodimers, and structurally similar to the EGF receptor binding domain described in the following references: WO 00/64400, Holmes et. al., Science,256:1205-1210 (1992), U.S. Pat.5,530,109 and 5,716,930, Hijazi et. al., Int.J.Oncol.,13:1061-1067 (1998), Chang et. al., Nature, 387:509-512 (1997), Carraway et. al., Nature, 387:512-516 (1997), Higashiyama et. al., J.Biochem.,122:675-680 (1997), and WO 97/09425. In some embodiments, the EGF-like domain binds to and activates ErbB2/ErbB4 or ErbB2/ErbB3 heterodimer. In some embodiments, the EGF-like domain comprises the amino acids of the receptor binding domain of NRG-1. In some embodiments, the EGF-like domain refers to the amino acids at Position 177-226, Position 177-237 or Position 177-240 of NRG-1. In some embodiments, the EGF-like domain comprises the amino acids of the receptor binding domain of NRG-2. In some embodiments, the EGF-like domain comprises the amino acids of the receptor binding domain of NRG-3. In some embodiments, the EGF-like domain comprises the amino acids of the receptor binding domain of NRG-4. In some embodiments, the EGF-like domain comprises the following amino acid sequence described in U.S. Pat.5,834,229: Ala Glu Lys Glu Lys Thr Phe Cys Val Asn Gly Gly Glu Cys Phe Met Val Lys Asp Leu Ser Asn Pro.

The dosage form, dose and route of administration of Neuregulin protein, and better pharmaceutical composition forms, can be determined according to methods known in the art (see, for example, Remington:The Science and Practice of Pharmacy, Alfonso R. Gennaro (Editor) Mack Publishing Company, 1997.4; Therapeutic Peptides and Proteins: Formulation, Processing,and Delivery Systems, Banga, 1999; Pharmaceutical Formulation Development of Peptides and Proteins, Hovgaard and Frkjr (Ed.),Taylor & Francis,Inc., 2000; Medical Applications of Liposomes, Lasic and Papahadjopoulos (Ed.), Elsevier Science, 1998, Textbook of Gene Therapy, Jain,Hogrefe & Huber Publishers, 1998; Adenoviruses: Basic Biology to Gene Therapy,Vol.15,Seth, Landes Bioscience,1999; Biopharmaceutical Drug Design and Development,Wu-Pong and Rojanasakul (Ed.), Humana Press,1999; Therapeutic Angiogenesis: From Basic Science to the Clinic, Vol. 28, Dole et al (Ed.), SpringerVerlag New York,1999).

Neuregulin protein can be prepared into oral, rectal, topical, inhalational, buccal (sublingual), parenteral (e.g., subcutaneous, intramuscular, intradermal or intravenous), percutaneous or other appropriate administration routes. Of all these modes of administration, the most appropriate one can be determined depending on the nature and severity of disease, and the properties of the special Neuregulin protein to be used. Neuregulin protein can be administered alone; or, more appropriately, co-administered with drug compatible carriers or excipients. Any appropriate drug acceptable carriers or excipients currently can be used in the current method (see, for example, Remington: The Science and Practice of Pharmacy,Alfonso R.Gennaro (Editor) Mack Publishing Company,April 1997).

According to the present invention, Neuregulin protein alone or a variety of its products made with other media, carriers, excipients could be used for numerous appropriate routes of administration, such as intracavemous injection, subcutaneous injection, intravenousinjection, intramuscular injection, intraderma injection, oral or buccal administration. It can be administered in unit dosage forms in ampouls or multi-dose containers with the addition of preservatives. It can be made into dosage forms such as suspension, solution, aqueous or butyraceous emulsion, and suspending agents, stabilizers and/or dispersants may be added; or, the active ingredient may be in powder form, before use, is dissolved with an appropriate medium, such as sterile and nonpyrogenic water or other solvents. The drug of the present invention when used as a topical product can be prepared into a foamable composition, gel, plaster, transdermal patch or pasta, etc.

For the drug of the present invention, the acceptable components and modes of administration include but are not limited to those published in U.S. Pat.5,736,154, 6,197,801B1, 5,741,511, 5,886,039, 5,941,868, 6,258,374B1, and 5,686,102.

The magnitude of a therapeutic dose in the treatment or prevention will vary with the severity of the condition to be treated and the route of administration. The dose, and perhaps dose frequency, will also vary according to age, body weight, condition and response of the individual patient.

It is noteworthy that the attending physician ought to know when and how to terminate or interrupt treatment or adjust/reduce dosage in case of toxicity or adverse reactions. Vice versa, if clinical response is unsatisfactory, the doctor should also know when and how to adjust the dose to high level.

Any appropriate route of administration can be used. The drug can be made into such dosage forms as tablet, lozenge, cachet, dispersion, suspension, solution, capsule, patch, etc, see, Remington's Pharmaceutical Sciences. In practical use, the neuregulin protein, alone or in combination with other agents, may be combined as the active in intimate admixture with a pharmaceutical carrier or excipient, such as beta-cyclodextrin and 2-hydroxy-propyl-beta-cyclodextrin, according to conventional pharmaceutical compounding techniques. The carrier may take a wide form of preparation desired for administration, topical or parenteral. In preparing compositions for parenteral dosage form, such as intravenous injection or infusion, similar pharmaceutical media may be employed, water, glycols, oils, buffers, sugar, preservatives, liposomes, and the like known to those of skill in the art. Examples of such parenteral compositions include, but are not limited to dextrose 5% wlv, normal saline or other solutions. The total dose of the neuregulin protein, alone or in combination with other agents to be administered may be administered in a vial of intravenous fluid, ranging from about 1 ml to 2000 ml. The volume of dilution fluid will vary according to the total dose administered.

The present invention also provides a kit for the implementation of the therapeutic regimen. The kit includes one or more containers of Neuregulin protein at effective dose. The protein can be provided alone or combined with other acceptable drugs/substances to form a composition. Preferred pharmaceutical forms would be in combination with sterile saline, dextrose solution, or buffered solution, or other pharmaceutically acceptable sterile fluid. Alternatively, the composition may be lyophilized or desiccated; in this instance, the kit optionally further comprises in a container a pharmaceutically acceptable solution, preferably sterile, to reconstitute the complex to form a solution for injection purposes. Exemplary pharmaceutically acceptable solutions are saline and dextrose solution,

The "therapy" or "treatment" herein refers to any manner in which the symptoms of a condition, disorder or disease are ameliorated or otherwise beneficially altered. The effect may be prophylactic in terms of completely or partially preventing a disease or symptom thereof and/or may be therapeutic in terms of a partial or complete cure for a disease and/or adverse effect attributable to the disease. Treatment also encompasses any pharmaceutical use of the compositions herein.

As used herein, "heart failure" means an abnormality of cardiac function where the heart does not pump blood at the rate needed for the requirements of metabolizing tissues. Heart failure includes a wide range of disease states such as congestive heart failure, myocardial infarction, tachyarrhythmia, familial hypertrophic cardiomyopathy, ischemic heart disease, idiopathic dilated cardiomyopathy, myocarditis and the like. The heart failure can be caused by any number of factors, including, without limitation, ischemic, congenital, rheumatic, viral, toxic or idiopathic forms. Chronic cardiac hypertrophy is a significantly diseased state which is a precursor to congestive heart failure and cardiac arrest.

Unless otherwise specified in the context, "protein" and "polypeptide", "peptide" used here have the same meaning.

Unless otherwise specified in the context, "plasma" and "serum" used herein have identical meaning.

Unless otherwise specified in the context, "not more than" used herein refers to "less than or equal to".

As used herein, "long-term benefit" means benefit caused by a treatment or interference which may not be observed in a short period after the treatment or interference. For chronic heart failure patients, long-term benefit may be improvement of survival, reduction of re-hospitalization or improvement of biomarkers which indicate the long-term prognosis. In some embodiments, the time period for observation of the benefit is about 6 months. In some embodiments, the time period for observation of the benefit is about 1 year. In some embodiments, the time period for observation of the benefit is about 2 years. And in other embodiments, the time period for observation of the benefit is about 3 years, 5 years, 10 years or longer.

As used herein, "survival" means the time or probability one subject may remain alive or living. It could be expressed by survival time or survival rate. Survival time is the time period start from the diagnosis or treatment to the end of the life. Survival rate means the percentage of people who are alive for a given period of time after diagnosis or treatment. For each subject, prolonged survival time caused by a treatment or interference could be regarded as a benefit. For a group of subjects or large populations, prolonged mean survival time or increased survival rate could be regarded as a benefit.

As used herein, "re-hospitalization" means the times or frequency of the patient admitted to the hospital in a given period of time. The admission to the hospital may be caused by all conditions, or only caused by the same condition which is being treated. For each subject, a reduction of times of re-hospitalizations in a given period of time could be regarded as a benefit. And for a group of subjects or large populations, a reduction of total times or mean times of re-hospitalizations could be regarded as a benefit.

As used herein, "N-terminal brain natriuretic peptide" or "NT-proBNP" means the inactive remnant N-terminal proBNP, the latter is the pro hormone of BNP which is a hormonally active natriuretic peptide that is mainly released from the cardiomyocytes in the left ventricular wall. In reaction to stretch and tension of the myocardial wall the pro hormone proBNP splits into BNP and the hormonally inactive remnant NT-proBNP by proteolytic cleavage.

BNP and NT-proBNP plasma levels are promising tools in the daily management of suspected or established heart failure. Most studies on the use of BNP and NT-proBNP in clinical practice addressed their diagnostic properties, and an increasingly amount of evidence is available supporting the prognostic value of BNP and NT-proBNP. As NT-proBNP has about 6 times longer of half-life in the blood than BNP, it is more widely used as a diagnostic or prognostic marker for heart failure. The plasma NT-proBNP level can be analyzed by commercial kits. For the purpose of example, but not limitation, the commercial kits from Roche or Biomedica.

Both BNP and NT-proBNP levels in the plasma are used for screening, diagnosis of heart failure and are useful to establish prognosis in heart failure, as both markers are typically higher in patients with worse outcome. And, it is discovered in the present invention that plasma level of BNP or NT-proBNP is indicative of the patient being suitable for heart failure treatment by neuregulin.

As used herein, "New York Heart Association (NYHA) Functional classification" or "NYHA" heart function classification is a simple way of classifying the extent of heart failure. It places patients in one of four categories based on how much they are limited during physical activity; the limitations/symptoms are in regards to normal breathing and varying degrees in shortness of breath and/or angina pain: I, no symptoms and no limitation in ordinary physical activity, e.g. shortness of breath when walking, climbing stairs etc.; II, mild symptoms (mild shortness of breath and/or angina) and slight limitation during ordinary activity; III, marked limitation in activity due to symptoms, even during less-than-ordinary activity, e.g. walking short distances (20-100 m), comfortable only at rest; and IV, severe limitations, experiences symptoms even while at rest, mostly bedbound patients.

As used herein, "activity unit" or "EU" or "U" means the quantity of standard product that can induce 50% maximal reaction. In other words, to determine the activity unit for a given active agent, the EC50 must be measured. For example, if the EC50 for a batch of product was 0.1 pg, which would be one unit. Further, if lpg of that product is being used, then 10 EU (1/0.1) is being used. The EC50 can be determined by any method known in the art, including the method employed by the inventors. This determination of the activity unit is important for quality control of genetically engineered products and clinically used drugs, permits product from different pharmaceuticals and/or different batch numbers to be quantified with uniform criteria.

The following is an exemplary, rapid, sensitive, high flux and quantitative method for determination of biological activity of NRG-1 through combining NRG with cell surface ErbB3/ErbB4 molecule and indirect mediation of ErbB2 phosphorylation (See e.g., Michael D. Sadick et al., 1996, Analytical Biochemistry, 235:207-214 and WO03/099300).

Briefly, the assay, termed a kinase receptor activation enzyme-linked immunosorbant assay (KIRA-ELISA), consists of two separate microtiter plates, one for cell culture, ligand stimulation, and cell lysis/receptor solubilization and the other plate for receptor capture and phosphotyrosine ELISA. The assay was developed for analysis of NRG-induced ErbB2 activation and utilizes the stimulation of intact receptor on the adherent breast carcinoma cell line, MCF-7. Membrane proteins are solubilized via Triton X-100 lysis and the receptor is captured in ELISA wells coated with ErbB2-specific antibodies with no cross-reaction to ErbB3 or ErbB4. The degree of receptor phosphorylation is then quantified by antiphosphotyrosine ELISA. A reproducible standard curve is generated with a EC50 of approximately 360pM for heregulin beta 1 (177-244). When identical samples of HRG beta 1 (177-244) are analyzed by both the KIRA-ELISA and quantitative antiphosphotyrosine Western Blot analysis, the results correlate very closely with one another. The assay described in this report is able to specifically quantify tyrosine phosphorylation of ErbB2 that results from the interaction of HRG with ErbB3 and/or ErbB4.

Since most of the genetically engineered medicines are proteins and polypeptides, their activity can be determined by their amino acid sequences or the activity center formed by their spatial structure. Activity titer of protein and polypeptide is not consistent with their absolute quality, therefore cannot be determined with weight unit as that of chemical drugs. However, biological activity of genetically engineered medicines is generally consistent with their pharmacodynamics and titer determination system established through given biological activity can determine its titer unit. Therefore, biological activity determination can be part of a titration process of the substance with biological activity and is an important component of quality control of genetically engineered product. It is important to determine biological activity criteria for quality control of genetically engineered product and clinically used drugs.

Quantity of standard product that can induce 50% maximal reaction is defined as an activity unit (1 EU). Accordingly, product from different pharmaceuticals and of different batch numbers can be quantitated with uniform criteria.

### DETAILED DESCRIPTION OF THE INVENTION

### Embodiment 1: A randomized, double-blinded, multi-center, placebo controlled survival study of recombinant human neuregulin in patients with chronic heart failure based on standard treatment(Study 209). (Study 209)

In order to evaluate the efficacy of Recombinant Human Neuregulin (rhNRG-1, ) for Injection in the treatment of chronic heart failure, a phase II, double-blinded, multi-center, placebo controlled, standard treatment based study was carried out in multiple clinical centers in China. A total of 351 patients with NYHA Class III or Class IV chronic heart failure were enrolled and randomized to Placebo Group or rhNRG-1 Group (0.6µgkg). There was no significant difference between the groups in terms of demographics and background treatment. The patients were admitted to hospital for administration of the drug for 10 consecutive days as scheduled and discharged on D11. From the 3rd week to the 25th week, the patients were administered a weekly dose in outpatient department. Blood samples were collected from the patients before treatment and after each administration. Determination of NT-proBNP level in plasma by a core laboratory (with reagent kit from Biomedica). In Week 52 of the study, information on the patients' survival was collected.

### Investigational product:

Specification: rhNRG-1, 61 amino acid polypeptide comprises the EGF-like domain of Neuregulin-1 β2 isoform, with the molecular weight of 7054 Dal (lpg=0.14nmol).

### Placebo:

Specification:Excipient for rhNRG-1 (250pg/vial without active recombinant human neuregulin-1 protein).

### Dosage regimen:

| | 1-10 days | 3-25 weeks |
|---|---|---|
| Dose | 0.6µgkg/day rhNRGl or placebo | 0.8µg/day rhNRG-1 or placebo |
| Route of administration | Intravenous drop | Intravenous infusion |
| Treatment course | 10 hours/day, for 10 consecutive days | weekly 10-minute infusion |

Criteria for participation in the trial included patients with CHF (NYHA class III or IV between the ages of 18 and 65 years old, LVEF≤40%, in relatively stable clinical condition (including clinical signs, symptoms and accepted standard treatment for CHF at the target dose or maximum tolerance dose for over 1 month). Major exclusion criteria included acute myocardial infarction, hypertrophic cardiomyopathy, constrictive pericarditis, significant valve disease or congenital heart disease, severe pulmonary hypertension, systolic blood pressure <90mmHg or >160mmHg, severe ventricular arrhythmia, cardiac surgery or a cerebrovascular event within the previous six months, claustrophobia or pregnant female subjects. All patients provided witnessed written consent.

### Embodiment 2: A multi-center, randomized, double-blinded, placebo controlled survival study of recombinant human neuregulin in patients with chronic heart failure based on standard treatment(Study301).

In order to evaluate the efficacy of Recombinant Human Neuregulin (rhNRG-1, ) for Injection in the treatment of chronic heart failure, a phase III, double-blinded, multi-center, placebo controlled, standard treatment based study was carried out in multiple clinical centers in China. A total of 331 patients with NYHA Class III or Class IV chronic heart failure were enrolled and randomized to Placebo Group or rhNRG-1 Group (06µg/kg) or rhNRG-1 Group (1.0µg/kg). There was no significant difference between the groups in terms of demographics and background treatment. The patients were admitted to hospital for administration of the drug for 10 consecutive days as scheduled and discharged on D11. From the 3rd week to the 25th week, the patients were administered a weekly dose in outpatient department. Blood samples were collected from the patients before treatment and after each administration. Determination of NT-proBNP level in plasma by a core laboratory (with reagent kit from Biomedica). In Week 52 of the study, information on the patients' survival was collected.

### Investigational product:

Specification: rhNRG-1, 61 amino acid polypeptide comprises the EGF-like domain of Neuregulin-1 β2 isoform, with the molecular weight of 7054 Dal (lpg=0.14nmol).

### Placebo:

Specification:Excipient for rhNRG-1 (250pg/vial without active recombinant human neuregulin-1 protein).

### Dosage regimen:

| | 1-10 days | 3-25 weeks |
|---|---|---|
| Dose | 0.6µg/kg/day rhNRGl or 1.0µg/kg/day rhNRG-1 or placebo | 0.8µg/kg/day rhNRG-1 or placebo |
| Route of administration | Intravenous drip | Intravenous infusion |
| Treatment course | 10 hours/day, for 10 consecutive days | weekly 10-minute infusion |

Criteria for participation in the trial included patients with CHF (NYHA class III or IV) between the ages of 18 and 80 years old, LVEF≤40%, in relatively stable clinical condition (including clinical signs, symptoms and accepted standard treatment for CHF at the target dose or maximum tolerance dose for over 1 month). Major exclusion criteria included acute myocardial infarction, hypertrophic cardiomyopathy, constrictive pericarditis, significant valve disease or congenital heart disease, severe pulmonary hypertension, systolic blood pressure <90mmHg or >160mmHg, severe ventricular arrhythmia, cardiac surgery or a cerebrovascular event within the previous six months, claustrophobia or pregnant female subjects. All patients provided witnessed written consent.

### Embodiment 3: a multi-center, randomized, double-blind, placebo controlled survival study of recombinant human neuregulin in patients with chronic heart failure based on standard treatment(Study 305)

In order to evaluate the efficacy of Recombinant Human Neuregulin (rhNRG-1, ) for Injection in the treatment of chronic heart failure, a double-blind, multi-center, placebo controlled, standard treatment based study was carried out in multiple clinical centers in China. A total of 679 patients with NYHA Class II or Class III chronic heart failure were enrolled and randomized to Placebo Group or rhNRG-1 Group (0.6µg/kg). There was no significant difference between the groups in terms of demographics and background treatment. The patients were admitted to hospital for administration of the drug for 10 consecutive days as scheduled and discharged on D11. From the 3rd week to the 25th week, the patients were administered a weekly dose in outpatient department. Blood samples were collected from the patients before treatment and after each administration. Determination of NT-proBNP level in plasma by a core laboratory (with reagent kit from Biomedica). In Week 52 of the study, information on the patients' survival was collected.

### Investigational product:

Specification: rhNRG-1, 61 amino acid polypeptide comprises the EGF-like domain of Neuregulin-1 β2 isoform, with the molecular weight of 7054 Dal (lpg=0.14nmol).

### Placebo:

Specification:Excipient for rhNRG-1 (250pg/vial without active recombinant human neuregulin-1 protein).

### Dosage regimen:

| | 1-10 days | 3-25 weeks |
|---|---|---|
| Dose | 0.6µg/kg/day rhNRG-1 or placebo | 0.8µg/kg/day rhNRG-1 or placebo |
| Route of administration | Intravenous drip | Intravenous infusion |
| Treatment course | 10 hours/day, for 10 consecutive days | weekly 10-minute infusion |

Inclusion criteria for the clinical study: chronic HF patients (NYHA Functional Class II or Class III) aged 18-75 yo, LVEF≤40%, clinical symptoms stable (including clinical symptoms, physical signs), having received HF basic standard of care at target dose level or maximum tolerated dose for more than 1 month). Exclusion criteria: acute myocardial infarction, hypertrophic cardiomyopathy, stenosing pericarditis, notable valvular pathological changes or congenital cardiac diseases, severe pulmonary artery hypertension, systolic pressure <90mmHg or >160mmHg, serious ventricular arrhythmia, patients received cardiac surgical treatment or experienced cerbrovascular accident in the 6 months prior to the study, claustrophobia or pregnant females. All patients need to understand and sign the Informed Consent Form.

The applicant performed statistical analyses of the data from the three separate clinical studies. Results from the analyses of pooled data from three separate clinical studies in all female subjects (mild, moderate, severe patients in NYHA Class II-IV): females in treatment groups achieved statistically significant survival benefits (P=0.016) vs. Patients in standard of treatment-based placebo-controlled group. In the study period, subjects' all-cause mortality decreased by 50% or above. In each of the separate studies, all females in treatment groups achieved survival benefits, showing a trend in agreement with that from the analysis of pooled data from the three studies.

Results from analysis of pooled data: generally speaking, during the study period, the lower the subjects' baseline NT-proBNP level, the greater their survival benefits (relative decrease of all-cause mortality); of the female subjects who had NT-proBNP level not more than 3000fmol/mL at baseline, those in treatment groups had a mortality rate of 0%; in contrast, the all-cause mortality of subjects in standard of treatment-based placebo-controlled group was nearly 10%; subjects in treatment groups achieved statistically very significant survival benefits (P<0.001). In all of the separate studies, female subjects showed a trend for survival benefits in agreement with that in the analysis of pooled data.

Clinical results revealed that rhNRG-1 could identify patients with mild to moderate heart failure more effectively, and this was in line with the results from studies in HF rat models induced by coarctation of aorta. It was shown when HF proceeded to a more serious stage when the heart deflects and blood pumping function collapse the expression of ErbB2 and ErbB4 receptors would decrease significantly. In light of this, at early stage of HF, i.e., before the downregulation of ErbB2/ErbB4 receptor expression, recombinant human Neuregulin can deliver more benefits to patients.

**Table 1. All-cause mortality - Summary of subgroup analysis: (Pooled analysis of data from Study 209/301/305)**

| Variable | | n/N (%) | |
|---|---|---|---|
| | | rhNRG-1 | Placebo |
| **Overall** | | 80/728(11.0) | 77/633(12.2) |

| **Gender** | | | |
|---|---|---|---|
| | Male | 68/560(12.1) | 53/484(11.0) |
| | Female | 12/168(7.1) | 24/149(16.1) |

| **NYHA Class at baseline** | | | |
|---|---|---|---|
| | Class II | 9/138(6.5) | 10/137(7.3) |
| | Class III | 46/501(9.2) | 59/441(13.4) |
| | Class IV | 25/89(28.1) | 8/55(14.5) |

| **NT-proBNP at baseline** | | | |
|---|---|---|---|
| | ≤1600 fmol/ml | 7/349(2.0) | 21/332(6.3) |
| | 1600-4000 fmol/ml | 37/238(15.5) | 29/190(15.3) |
| ≥4000 fmol/ml | | 30/95(31.6) | 22/81(27.2) |
| Skipping | | 6/46(13.0) | 5/30(16.7) |

| | | | |
|---|---|---|---|
| Abbreviations: LVEF=left ventricular ejection fraction, N=total number of subjects analyzed, n =number of subjects in a specific category, NYHA=New York Heart Association | | | |

When assay results revealed that baseline NT-proBNP was ≤1600fmol/ml, rhNRG-1 showed the potential to lower the mortality of subjects significantly (Table 1) regardless of subject's sex; however, this conclusion does not apply to the analysis of pooled data from subjects of both sexes who have higher baseline NT-proBNP. Further analysis revealed that rhNRG-1 had resulted in statistically significantly lower mortality (Table 1) in all test female subjects. It was found in Individual Studies 209, 301 and 305 that females showed the same trend, as shown in Table 2.

**Table 2. All-cause mortality - Summary of subgroup analysis: Single clinical study (Analysis of Study 209/301/305)**

| | 209 | | 301 | | 305 | |
|---|---|---|---|---|---|---|
| | n/N(%) | | n/N(%) | | n/N(%) | |
| Variable | rhNRG-1 | Placebo | rliNRG-1 | Placebo | rhNRG-1 | Placebo |
| **Overall Gender** | 18/175(10.3) | 29/176(16.5) | 34/214(15.9) | 14/117(12.0) | 28/339(8.3) | 34/340(10.0) |
| Male | 14/142(9.9) | 18/138(13.0) | 32/168(19.0) | 10/86(11.6) | 22/250(8.8) | 25/260(9.6) |
| Female | 4/33(12.1) | 11/38(28.9) | 2/46(4.35) | 4/31(12.9) | 6/89(6.7) | 9/80(11.3) |

| | | | | | | |
|---|---|---|---|---|---|---|
| Abbreviations: LVEF=left ventricular ejection fraction, N=total number of subjects analyzed, n =number of subjects in a specific category, NYHA=New York Heart Association | | | | | | |

Baseline NT-proBNP percentiles were subjected to further subgroup analysis for female subjects since they showed better improvements in both pooled studies and individual studies. These subgroups were subjected to pooled and single analyses for patients whose NT-proBNP≤1600fmol/ml (~50%), NT-proBNP≤3000fmol/ml (~75%) and all female patients, as shown in Table 3.

Pooled analysis of the three clinical studies 209, 301 and 305 revealed that the mortality rate of females subjects was 0%, 0% and 7.1%, respectively, in the three subgroups of female subjects on rhNRG-1 treatment, i.e., Subgroup NT-proBNP≤1600, Subgroup NT-pro BNP≤3000fmol/ml, and all female patients on the therapy. The mortality rate in the three corresponding subgroups of subjects on placebo was 8.4%, 10.4% and 16.1%, respectively; it was shown that rhNRG-1 can significantly lower mortality rate, particularly in the NT-proBNP≤3000fmol/ml subgroup, female subjects in this subgroup account for 75% of the enrolled female subjects, as shown in Table 3.

Individual analyses of studies 209, 301 and 305 revealed that the mortality rate of female subjects in the NRG-1 Treatment Group was 0%, 0% and 0%, respectively, for NT-proBNP≤3000fmol/ml subgroups in the studies, while the mortality rate was 21.4%, 8.3% and 6.3%, in the corresponding placebo group, as shown in Table 3.

For male patients, in NT-proBNP≤4000fmol/ml or NT-proBNP≤3000fmol/ml subgroups, some clinical studies showed rhNRG-1 Treatment Group had mortality rate superior to that in placebo group. More preferably, pooled analyses of male subjects whose NT-proBNP≤1600fmol/ml in studies 209, 301 and 305 revealed that the mortality rate in rhNRG-1 Treatment Group was substantially lower than that in the Placebo group (2.6%vs 5.6%). This decrease was in line with the comprehensive analysis of all subjects of both sexes.

**Table 3. All-cause mortality - Summary of female subgroup analysis: (Pooled analysis & Individual analysis of data from Study 209/301/305)**

| **All-cause mortality** | **Baseline NT-proBNP (fmol/ml)** | **NT-proBNP value-based female subgroup** | |
|---|---|---|---|
| Clinical studies | | rhNRG-1 n/N (%) | Placebo n/N (%) |
| **209** | ≤1600 | 0/12 (0.0) | 3/20 (15.0) |
| | ≤3000 | 0/16 (0.0) | 6/28 (21.4) |
| | All | 4/33 (12.1) | 11/38 (28.9) |
| **301** | ≤1600 | 0/16 (0.0) | 2/20 (10.0) |
| | ≤3000 | 0/33 (0.0) | 2/24 (8.3) |
| | All | 2/46 (4.3) | 4/31 (12.9) |
| **305** | ≤1600 | 0/53 (0.0) | 2/43 (4.7) |
| | ≤3000 | 0/69 (0.0) | 4/63 (6.3) |
| | All | 6/89 (6.7) | 9/80 (11.3) |
| **209, 301 & 305** | ≤1600 | 0/81 (0.0) | 7/83 (8.4) |
| | ≤3000 | 0/118 (0.0) | 12/115 (10.4) |
| | All | 12/168 (7.1) | 24/149 (16.1) |

In light of the above analyses, individual and pooled analyses of the female subjects who had NT-proBNP≤3000fmol/ml and male subjects who had NT-proBNP≤1600fmol/ml in any of the three clinical studies were performed as shown in Table 4. For these pooled male and female subjects, the mortality improvement result in the rhNRG-1 Treatment Group was significantly superior to that in the placebo group.

**Table 4. All-cause mortality - Summary of subgroup analysis: (Pooled analysis & Individual analysis of data from Study 209/301/305)**

| **All-cause mortality** | **Female NT-proBNP≤3000 & Male NT-proBNP ≤ 1600 fmol/ml** | |
|---|---|---|
| **Clinical studies** | rhNRG-1 n/N (%) | Placebo n/N (%) |
| **209** | 1/72 (1.4) | 10/91 (11.0) |
| **301** | 1/106 (0.9) | 2/63 (3.2) |
| **305** | 5/208 (2.4) | 14/210 (6.7) |
| **209, 301&305** | 7/386 (1.8) | 26/364 (7.1) |

The above-listed embodiments would not limit the protection range of the study. Technicians in the art may adjust and modify this invention without deviation from the purpose and scope of the invention. Therefore, the protection range of this invention should be defined according to rights and requirements rather than by specific embodiments.

## Claims

1. Use of NRG in the manufacture of a medicament for the treatment of heart failure (HF) in a patient, wherein the patient is a female HF patient whose pre-treatment plasma level of NT-proBNP is not more than 3000fmol/ml.

2. The use of Claim 1, wherein the NRG is NRG-1.

3. The use of Claim 1, wherein the NRG comprises an EGF-like domain of NRG-1.

4. The use of Claim 1, wherein the NRG comprises an amino acid sequence of SEQ ID NO:1

5. A method of screening a HF patient suitable for treatment with Neuregulin, comprising performing a pre-treatment diagnostic test, and determining the eligibility of the patient for Neuregulin treatment according to the test results.

6. The method of Claim 5, wherein the diagnostic test is a test for the plasma level of NT-proBNP or BNP.

7. The method of Claim 6, when a female HF patient has a pre-treatment plasma level of NT-proBNP not more than 3000fmol/ml, the diagnostic test results indicate suitability for treatment with Neuregulin.

8. A diagnostic kit for screening a HF patient suitable for treatment with Neuregulin, wherein the diagnostic kit comprises immunoassay reagents for measuring the NT-proBNP plasma level in HF patients.

9. The diagnostic kit of Claim 8, when a female HF patient has a pre-treatment plasma level of NT-proBNP not more than 3000fmol/ml, the diagnostic test results indicate suitability for treatment with Neuregulin.

10. A kit for the treatment of heart failure, comprising the diagnostic kit of Claim 8 and an effective dose of NRG.
